# FASCICULE DE BREVET EUROPEEN

(11) **EP 2 217 255 B1**
(45) Date de publication et mention de la délivrance du brevet: **03.07.2019**
(21) Numéro de dépôt: 08856497.6
(22) Date de dépôt: 19.11.2008
(51) Int. Cl.: A61K 31/56, A61K 36/21, A61P 3/04

(54) **UTILISATION DE PHYTOECDYSONES DANS LA PREPARATION D'UNE COMPOSITION POUR AGIR SUR LE SYNDROME METABOLIQUE**
VERWENDUNG VON PHYTOECDYSONEN BEI DER HERSTELLUNG EINER ZUSAMMENSETZUNG ZUR EINWIRKUNG AUF DAS STOFFWECHSELSYNDROM
USE OF PHYTOECDYSONES IN THE PREPARATION OF A COMPOSITION FOR ACTING ON METABOLIC SYNDROME

(30) Priorité: 30.11.2007 FR 0759478
(43) Date de publication de la demande: 18.08.2010
(73) Titulaire: Biophytis, 75001 Paris (FR); Sorbonne Université, 75006 Paris (FR); Centre National de la Recherche Scientifique (C.N.R.S.), 75794 Paris Cedex 16 (FR)
(72) Inventeur: VEILLET, Stanislas, F-91600 Savigny sur Orge (FR); LAFONT, René, F-75016 Paris (FR)
(74) Mandataire: Ipside
(86) Numéro de dépôt international: PCT/FR2008/052088
(87) Numéro de publication internationale: WO 2009/071804

(56) Documents cités:
- WO-A1-2004/016092
- WO-A2-2007/133473
- GB-A- 2 420 066
- DATABASE WPI Week 1992 Thomson Scientific, London, GB; AN 1992-189214 XP002483882 "New steroidal insect metamorphosis hormone, e.g. alpha-ecdysone, inokosterone and pterosterone, for treaing diabetes." & JP 04 124135 A (VERITAS CORP.) 24 avril 1992 (1992-04-24) cité dans la demande
- DATABASE WPI Thomson Scientific, London, GB; AN 2005-243149 XP002483883 "Use of ecdysteronein preparation of insulin resistance medicine." & CN 1 557 324 A (CHEN Q.) 29 décembre 2004 (2004-12-29) cité dans la demande
- DATABASE TCM-SIPO SIPO; CN1280010, Kunming Pharmaceutical Co. Ltd. 17 octobre 2001 (2001-10-17), YANG C.: "An oral medicine for the treatment of diabetes and its preparation method." XP002483879 cité dans la demande
- ZHU N. ET AL.: "Ecdysteroids of quinoa seeds (Chenopodium quinoa Willd.)" J. AGRIC. FOOD CHEM., vol. 49, 2001, pages 2576-2578, XP002483876 cité dans la demande
- KUTEPOVA T.A. ET AL.: "Hypoglycemic activity of the total ecdysteroid extract from Ajuga turkestanica." PHARM. CHEM. J., vol. 35, no. 11, 2001, pages 608-609, XP002483877
- DATABASE CA CHEMICAL ABSTRACTS SERVICE, COLUMBUS, OHIO, US; Doklady Akad. Nauk Respub. Uzbekistan (4) 46-49 1997, SYROV V.N. ET AL.: "Hypoglycemic action of phytoecdysteroids and somemechanisms of its realisation in experimental animals." XP002483880
- DATABASE BIOSIS BIOSCIENCES INFORMATION SERVICE, PHILADELPHIA, PA, US; Voprosy Meditsinskoi Khimii,vol.28 pages 101-105 1982, MIRONOVA V.N. ET AL.: "Hypocholesterolemic action of phytoecdysones in experimental hypercholesterolemia in rats." XP002483881 cité dans la demande
- LAFONT R. ET DINAN L.: "Practical use of ecdysteroids in mammals including humans :an update." J. INSECT SCI., vol. 3, no. 7, 2003, pages 1-30, XP002483878 cité dans la demande
- CATALAN R E ET AL: "Alterations in rat lipid metabolism following ecdysterone treatment" COMPARATIVE BIOCHEMISTRY AND PHYSIOLOGY. B. COMPARATIVEBIOCHEMISTRY, PERGAMON PRESS, LONDON, GB, vol. 81, no. 3, 1 janvier 1985 (1985-01-01), pages 771-775, XP023529585 ISSN: 0305-0491 [extrait le 1985-01-01]
- DINAN L ET AL: "On the distribution of phytoecdysteroids in plants", CMLS CELLULAR AND MOLECULAR LIFE SCIENCES, BIRKHAUSER VERLAG, HEIDELBERG, DE, vol. 58, no. 8, 1 July 2001 (2001-07-01), pages 1121-1132, XP007912150, ISSN: 1420-682X, DOI: 10.1007/PL00000926

## Description

### Domaine technique

L'invention concerne l'utilisation de phytoecdysones, apportées pures ou sous forme d'extrait, pour la préparation d'une composition destinée à prévenir ou traiter le syndrome métabolique chez le mammifère. Plus particulièrement, l'utilisation selon l'invention permet de diminuer la masse grasse et/ou de réduire son accumulation notamment au niveau de la ceinture abdominale. L'invention concerne également un extrait de plantes enrichi en phytoecdysones qui peut être utilisé pour la préparation d'une telle composition. L'invention peut avantageusement être utilisée dans le domaine de l'agroalimentaire, afin d'enrichir des produits alimentaires en phytoecdysones pour en faire des aliments fonctionnels, ou alicaments. L'invention trouve également des applications dans le domaine médical, les phytoecdysones étant, par exemple, utilisées dans une composition médicinale.

### Etat de la technique

La surcharge pondérale est de nos jours un état physiologique très répandu, et ce quelle que soit la classe d'âge et la culture. Ce désordre physiologique peut être à l'origine de nombreuses complications de santé. Par exemple, le syndrome métabolique est une perturbation physiologique le plus souvent liée chez un individu au surpoids. On reconnaît qu'une personne est atteinte du syndrome métabolique lorsqu'elle présente au moins trois des cinq signes cliniques associés à cet état, à savoir une obésité viscérale ou abdominale, une hypertriglycéridémie, une dyslipidémie athérogène, une hypertension et une hyperglycémie (Isomaa et al., 2001). L'obésité augmente par ailleurs en elle-même les risques de développer un diabète de type II, ou diabète gras, et des maladies cardio-vasculaires (Rexrode et al., 1998).

De nombreux médicaments, tels que des médicaments hypotenseurs et hypocholestérolémiants ont été développés pour contrer le développement de maladies cardiovasculaires chez les individus à risque (Foster-Schubert et al, 2006). De même, les diabétiques peuvent, lorsque la prescription d'un régime alimentaire pauvre en graisse et en sucre ne suffit plus, recevoir un apport en insuline.

Cependant, pour la plupart des individus atteints du syndrome métabolique, l'administration d'un cocktail de médicaments associée à la prescription d'un régime strict et à la nécessité de pratiquer un exercice physique régulier, est source de découragement, d'autant plus que les effets bénéfiques ne sont pas immédiats et que l'hygiène de vie imposée doit être maintenue, au risque sinon de voir les symptômes associés au syndrome métabolique réapparaître (Wasan et al., 2005).

Il est donc nécessaire que les individus présentant un syndrome métabolique puissent retrouver un équilibre physiologique leur permettant de ne plus être répertoriés comme à risque, notamment du point de vue du développement des maladies cardio-vasculaires, sans pour autant devoir supporter un traitement trop lourd et/ou trop contraignant. Il est également nécessaire d'éviter l'installation de tout ou partie des symptômes associés, qui peuvent conduire à la destruction de la masse maigre et au diagnostic de syndrome métabolique (Unger, 2003).

Une alimentation équilibrée complétée avec des aliments fonctionnels ou des compléments alimentaires permet d'agir sur l'état de santé du mammifère en surpoids et prévenir l'installation du syndrome métabolique. De nombreux compléments alimentaires ou aliments fonctionnels ont été développés pour favoriser la perte de poids chez le mammifère en surpoids (Saper et al., 2004) et prévenir le développement du diabète (McWorther, 2001) et de maladies cardiovasculaires. La plupart des produits alimentaires actuellement sur le marché font preuve d'une efficacité insuffisante pour agir sur l'obésité abdominale et, dans certains cas, ils se sont révélés être toxiques (Pittle et al., 2005). Il est donc nécessaire d'identifier de nouvelles molécules naturelles, déjà présentes dans l'alimentation des mammifères, pour développer des ingrédients et des aliments fonctionnels non toxiques et efficaces sur la masse grasse.

Les Phytoecdysones sont des molécules naturelles de la famille des triterpènes, relativement abondantes dans le règne végétal où elles sont présentes chez 5% des plantes sauvages (Báthori et Pongrács, 2005). Les effets physiologiques des phytoecdysones, en particulier de la 20-hydroxyecdysone chez les mammifères ont été étudiés par plusieurs équipes. Il a été montré en particulier que ces molécules stimulaient les synthèses protéiques (Otaka et al., 1968 ; Khimiko et al., 2000 ; Syrov, 2000) et la croissance animale (Purser et Baker, 1994). Des effets hypoglycémiants (Uchiyama et al., 1970 ; Mironova et Kholodova, 1982 ;Takahashi et Nishimoto, 1992 ; Yang et al., 2001 ; Chen et al., 2004, 2006), hypolipidémiants (Mironova et al., 1982 ; Syrov et al., 1983) et cholagogues (Syrov et al., 1986) ont également été rapportés. En outre, ces molécules présentent des propriétés antioxydantes (Kuzmenko et al., 2001) et sont dépourvues de toxicité

### Exposé de l'invention

Les inventeurs ont découvert que l'ingestion régulière ou non de phytoecdysones permet d'agir positivement au moins sur l'accumulation de masse grasse, principalement au niveau de la ceinture abdominale. Les phytoecdysones sont des ecdystéroïdes d'origine végétale. Par agir positivement, on entend que l'ingestion de phytoecdysones permet de réduire la masse grasse chez le mammifère déjà obèse, mais également permet de réduire la formation de la masse grasse alors même que le régime suivi devrait favoriser l'accroissement de la masse grasse, que le mammifère soit en croissance ou adulte. L'ingestion de phytoecdysones permet de diminuer la masse grasse, mais joue aussi sur l'hyperglycémie et la dyslipidémie athérogène, et de ce fait permet de prévenir ou de traiter le syndrome métabolique chez l'individu en surpoids ou obèse.

Un individu est considéré en surpoids dès lors que son indice de masse corporel (IMC) est supérieur à 25, et est considéré comme obèse à partir d'un IMC égal ou supérieur à 30.

L'invention propose donc d'apporter des phytoecdysones dans une composition destinée au mammifère, ou dans une composition médicinale, en vu de supprimer ou d'éviter l'apparition du syndrome métabolique. La diminution de la masse grasse déjà acquise, peut permettre chez certains individus de ne plus souffrir du syndrome métabolique. En effet, la diminution de la masse grasse joue de manière indirecte sur plusieurs des symptômes associés au syndrome métabolique, et notamment sur l'obésité abdominale, sur la glycémie et sur la dyslipidémie athérogène. De plus, cela peut également avoir une action préventive sur le développement du syndrome métabolique chez les personnes en surpoids, en permettant une diminution de la masse grasse déjà acquise et/ou en évitant une prise supplémentaire.

Plus généralement, l'utilisation de phytoecdysones selon l'invention permet d'éviter l'accumulation de masse grasse abdominale et de diminuer la masse grasse abdominale déjà acquise chez tout individu quel que soit son IMC, et dans le cas des individus en surpoids ou obèses, elle permet de prévenir et/ou traiter le syndrome métabolique.

Les phytoecdysones peuvent être apportées pures, ou sous forme d'extrait de plantes plus ou moins enrichi. Avantageusement, l'extrait de plantes enrichi en phytoecdysones selon l'invention provient d'un extrait de quinoa. En effet, le quinoa est une pseudo céréale comestible naturellement riche en phytoecdysones (Zhu et al., 2001 ; Dini et al., 2005). Il est ainsi possible de compléter l'alimentation par ingestion d'extrait de quinoa enrichi en phytoecdysones, en introduisant cet extrait dans un aliment, tel qu'un produit laitier ou une boisson, ou en le consommant, en tant que complément alimentaire, sous forme de gélules par exemple. Un des intérêts du quinoa est que c'est une plante entrant dans l'alimentation humaine au même titre que le blé ou le sarrasin, et non une plante médicinale dont on pourrait ignorer certains effets secondaires. Les inventeurs se sont particulièrement intéressés au quinoa non seulement pour ses qualités nutritionnelles mais également parce qu'il présente naturellement une quantité importante de phytoecdysones.

La capacité à produire des phytoecdysones semble perdue chez la grande majorité des espèces de plantes cultivées, et de ce fait l'alimentation humaine « classique » ne contient que de faibles quantités de phytoecdysones, soit en moyenne moins de 1 mg par jour. Le quinoa représente à ce jour la plante alimentaire de loin la plus riche en phytoecdysones. Les graines de quinoa contiennent un mélange de phytoecdysones (Zhu et al., 2001). Ces phytoecdysones sont particulièrement abondantes dans l'enveloppe des graines de quinoa. Par exemple, une ration de graines de quinoa de 60 grammes (poids sec) comprend entre 15 et 20 milligrammes de 20-hydroxyecdysone.

L'épinard et certains champignons peuvent également être avantageusement utilisés pour la fabrication d'un extrait de plantes riche en phytoecdysones (Findeisen E, 2004).

L'invention a donc pour objet une utilisation de phytoecdysones, pures ou contenues dans un extrait de plantes spécialement enrichi en phytoecdysones, pour la réalisation d'une composition destinée à diminuer la masse grasse abdominale chez le mammifère. Notamment, elle permet de diminuer la masse grasse abdominale déjà acquise. Elle permet également, dans le cas d'un régime hyperlipidique notamment, de diminuer l'accumulation de la masse grasse abdominale.

Selon l'invention, la composition comprenant des phytoecdysones est particulièrement destinée aux individus présentant un surpoids ou obèses, et permet de prévenir et/ou traiter le syndrome métabolique.

La composition ainsi préparée peut être consommée par une personne présentant une surcharge pondérale présentant un syndrome métabolique ou susceptible de développer un syndrome métabolique. La prise régulière de la composition selon l'invention favorise la diminution de la masse grasse, notamment au niveau de la ceinture abdominale. La surcharge pondérale graisseuse diminuant, les risques de complications diminuent également.

La composition s'entend par exemple d'un produit alimentaire tel qu'une boisson, un produit laitier ou autre. Bien entendu, la composition peut être une composition médicinale par exemple utilisée sous forme de pilules qui contiennent ainsi une dose bien précise de phytoecdysones. Par composition médicinale, on entend une composition présentant des propriétés curatives et/ou préventives vis-à-vis de l'accumulation de la masse grasse et indirectement du syndrome métabolique, dont la prise est soumise à un suivi médical, plus ou moins strict. L'avantage de la consommation sous forme de pilules est que le consommateur est en mesure d'évaluer de manière certaine la quantité de phytoecdysones consommée, et ce indépendamment de son appétit. Bien entendu, le fait d'associer la prise de phytoecdysones à la prise d'un aliment, c'est-à-dire de consommer les phytoecdysones sous forme de composition alimentaire, permet de dépasser la notion de traitement et donc de contraintes liées à un état clinique spécifique

Les phytoecdysones utilisées peuvent être obtenues par extraction, à partir de plantes contenant des phytoecdysones. Les phytoecdysones utilisées peuvent également être des phytoecdysones de synthèse. Dans le cas d'une extraction, l'extrait de plante utilisé est préférentiellement un extrait de plantes alimentaires telles que le quinoa. Par plantes alimentaires, on entend des plantes essentiellement utilisées comme aliment ou comme condiment sur une large zone géographique. Inversement, les plantes médicinales sont des plantes essentiellement utilisées pour leurs vertus curatives.

L'invention a également pour objet un extrait de plantes alimentaires enrichi en phytoecdysones. Avantageusement, ledit extrait comporte au moins 1% et préférentiellement entre 1% et 7%, de manière préférée entre 1.5% et 3% et encore plus préférentiellement 2% en poids de phytoecdysones.

Les plantes à partir desquelles sont réalisés les extraits selon l'invention sont avantageusement choisies parmi le quinoa, les épinards et les champignons.

L'invention a aussi pour objet une composition destinée à l'alimentation des mammifères, comportant de l'extrait de plantes enrichi en phytoecdysones.

### Brève description des figures

- Figure 1 : Graphique représentant le gain de poids en g/jour en fonction du régime auquel les souris sont soumises ;
- Figure 2 : Graphique représentant le pourcentage d'adipocytes épidydymaires (%TAE en mg/g BW) en fonction du régime auquel les souris sont soumises ;
- Figure 3 : Graphique représentant l'adiposité sous-cutanée (%TASC en mg/g BWT) en fonction du régime auquel les souris sont soumises ;
- Figure 4 : Graphique représentant le diamètre des adipocytes, en µm, en fonction du traitement administré aux souris soumises au régime Saindoux ;
- Figure 5 : Graphique montrant la distribution des diamètres des adipocytes en fonction du traitement administré aux souris soumises au régime Saindoux.

### Description détaillée

Dans l'invention, on propose d'apporter une dose concentrée de phytoecdysones pures, ou par le biais d'un extrait de plantes enrichi en phytoecdysones, afin d'améliorer l'état sanitaire de personnes en surcharge pondérale, ou d'éviter l'installation d'une surcharge pondérale par accumulation de masse grasse abdominale et viscérale. C'est un des marqueurs clinique fondamentaux du syndrome métabolique et un facteur de risque du développement des maladies cardio-vasculaires et du diabète de type 2.

La dose seuil de phytoecdysones contenue dans une composition selon l'invention et permettant d'obtenir des effets positifs sur la masse grasse est de 0,3 à 0,5 mg/kg chez l'homme soit de 20 à 30 mg par jour en moyenne. Cette estimation est basée sur la détection d'un effet physiologique chez la souris (Mus musculus lignée C57BI6) à une dose de 5 mg/kg. Cette dose a été ajustée d'un facteur de correspondance pharmacologique prenant en compte une demi-vie moyenne des phytoecdysones plus importante chez l'homme que chez la souris (Simon et Koolman, 1989).

Selon l'invention, il est possible d'apporter cette dose de phytoecdysones quotidienne sous la forme d'un extrait de plantes, tel que de quinoa, incorporé par exemple dans un aliment entrant dans l'alimentation courante d'un individu. En effet, dans 4 grammes d'extrait de quinoa enrichi à hauteur de 0,5% en poids de phytoecdysones, on a 20 milligrammes de phytoecdysones. Pour obtenir la même quantité de phytoecdysones à partir de graines de quinoa, il faudrait consommer de 50 à 100 grammes de graines de quinoa non traitées (Dini et al., 2005). L'extrait de quinoa selon l'invention peut ainsi contenir jusqu'à 50 fois plus de phytoecdysones que les graines de quinoa dont l'extrait est issu. Il est donc tout à fait envisageable, avec l'utilisation selon l'invention, de couvrir les besoins en phytoecdysones de l'individu, sans contraintes alimentaires, puisque l'ingestion d'un seul aliment de la vie courante, tel qu'un yaourt, peut apporter la dose souhaitée. L'extrait selon l'invention peut être ajouté au moment de l'élaboration d'une composition alimentaire, afin que l'aliment fini le contienne. Ainsi, certain aliments ou boissons vendus en grandes et moyennes surfaces pourront contenir une dose donnée de phytoecdysones. Il est également possible d'ajouter l'extrait enrichi en phytoecdysones à des céréales au moment de leur consommation, avant ou après cuisson. Dans ce cas, l'individu peut adapter la quantité de phytoecdysones à ses besoins propres.

Selon un exemple de l'invention, l'extrait de plantes enrichi en phytoecdysones est introduit dans des aliments de tous les jours, c'est-à-dire qui sont consommés dans un but avant tout nutritif, et non thérapeutique ou prophylactique.

En terme quantitatif, chaque individu peut consommer plusieurs centaines de kilos d'un même aliment par an. Si on ajoute à ce type d'aliments de consommation courante un extrait de plantes enrichi en phytoecdysones, les consommateurs peuvent recevoir une dose suffisante de phytoecdysones pour prévenir l'apparition d'une surcharge pondérale liée à une accumulation trop important de graisse notamment au niveau de la ceinture abdominale, ou l'aggravation de leur état, sans entrer dans une démarche de traitement. Cet extrait enrichi peut être considéré comme une substance « adaptogène » (Báthori M., Pongrács Z., 2005). Par substance adaptogène, on entend substance qui n'est pas un médicament mais qui améliore l'équilibre général pour rendre l'individu plus résistant ou plus apte à ne pas développer les maladies associées au syndrome métabolique en cas de surpoids.

L'avantage du quinoa est que c'est une plante facile à cultiver, ne nécessitant pas d'entretien particulier, qui a peu de besoin en eau et qui supporte des conditions de culture extrêmes.

De plus, les phytoecdysones issues du quinoa ne sont pas dégradées par la chaleur. Il est donc possible de préparer des compositions alimentaires destinées à être chauffées ou cuites avec un extrait de quinoa enrichi selon l'invention.

Bien entendu, les mêmes quantités de phytoecdysones peuvent, selon un autre exemple de l'invention, être apportées à un patient par ingestion d'une composition médicinale comportant la dose prescrite de phystoecdysones, la composition étant par exemple délivrée sous forme de gélules ou cachets.

### I] Exemples de procédé de préparation d'extrait de quinoa enrichi en phytoecdysones

### 1-Exemple : procédé A

On procède à une extraction séquentielle par l'eau, en ajoutant 500g de graines de quinoa à 2 L d'eau bouillante, et on maintient l'ensemble pendant 5 minutes à 80 °C. On élimine l'eau et on réalise une seconde extraction avec 2 L d'un mélange éthanol-eau (1 :1) sous agitation pendant 20 minutes à 80 °C.

Une telle extraction séquentielle permet de supprimer de l'extrait les saponines, abondantes dans les graines de quinoa (Muir et al., 2002), qui conféreraient un goût amer audit extrait.

L'extrait éthanolique est filtré sur miracloth™, évaporé à sec et repris avec 400 mL d'éthanol absolu, ce qui laisse un résidu insoluble abondant.

La fraction éthanolique est filtrée ou centrifugée puis séchée.

L'analyse chromatographique (HPLC) montre que cet extrait contient 2 ± 0,2 % en poids de 20-hydroxyecdysone.

### 2- Exemple : procédé B

On mélange 50 g de graines de quinoa à 400 mL d'eau distillée. Le mélange est introduit dans un micro-ondes pendant 5 mn à puissance moyenne (800 W).

On procède ensuite à une seconde extraction avec de l'éthanol (400 ml d'éthanol) pendant 2,5 mn au micro-ondes toujours à puissance moyenne (800 W).

Les deux procédés d'extraction permettent d'obtenir entre 150 et 200 milligrammes de phytoecdysones par kilogramme de graines de quinoa traitées, dont 85-90 % correspondent à la 20-hydroxyecdysone, et le restant à des ecdystéroïdes de structure très voisine.

### 3- Exemple: Procédé C pour enrichir l'extrait

Il est possible d'enrichir l'extrait du procédé A (ou B) par une partition butanol-eau, qui permet d'éliminer les composés les plus polaires, et d'aboutir après évaporation de la phase butanol à un extrait plus concentré en phytoecdysones (5-7% en poids).

### II] Etude expérimentale de l'effet des phytoecdysones sur le stockage de la masse grasse

### Protocole

On teste l'effet des phytoecdysones sur des souris soumises à un régime gras pendant 3 semaines.

Le régime gras ou régime inducteur consiste en l'apport de quantités importantes de matière grasse sous la forme de saindoux. Les souris choisies dans cette étude sont des souris C57 BL/6, mâles, âgées de 6 semaines lorsqu'elles commencent à suivre ce régime inducteur. De telles souris constituent un modèle d'étude privilégié pour analyser l'impact de l'extrait de quinoa enrichi en phytoecdysones selon l'invention sur les paramètres physiologiques du syndrome métabolique.

On teste également, en parallèle, l'effet de ces mêmes phytoecdysones sur des souris non soumises à un régime gras, constituant le régime normal témoin.

Le tableau 1 ci-dessous indique la répartition des souris étudiées en fonction des régimes et des traitements auxquels elles sont soumises.

**Tableau 1 : Répartition des souris étudiées :**

| | | **Traitements** | | | |
|---|---|---|---|---|---|
| | | **Témoin** | **Principe actif pur 20E** | **Extrait 1.6%** | **Extrait 7 %** |
| **Régimes** | **Inducteur** | 6 souris | 6 souris | 6 souris | 6 souris |
| | **Témoin** | 6 souris | 6 souris | 6 souris | 6 souris |

Le régime inducteur s'entend du régime enrichi en saindoux.

Le principe actif pur s'entend de 20-hydroxyecdysone (20E) pure.

Les 24 souris de chaque série sont soumises au régime tel que détaillé au tableau 2, pendant trois semaines et sont traitées en parallèle avec la molécule pure, ou un extrait de quinoa enrichi en 20-hydroxyecdysone (20E) à hauteur de 1,6 % (2,24 % la dernière semaine de traitement pour la série témoin) ou 7 %. La concentration en 20-hydroxyecdysone est ajustée à 40 mg par kg d'aliment.

Compte tenu de la quantité d'aliment ingérée en moyenne par les souris, la dose de 20E administrée correspond dans les 3 traitements à 5 mg de 20E par kg de poids corporel et par jour. L'aliment est fourni en excès, trois fois par semaine, pour les deux régimes et les 3 traitements. En moyenne, 40g d'aliment sont apportés par cage et par jour, soit 6,5g d'aliment par souris et par jour.

Le tableau 2 ci-dessous indique plus en détail la composition du régime alimentaire auquel les souris sont soumises

**Tableau 2 : Composition des régimes :**

| | Composition (g/kg) | |
|---|---|---|
| Ingrédients | P14 témoin | P14 saindoux |
| lait (LR85 F) | 140 | 170 |
| Amidon | 622,4 | 360 |
| saccharose | 100,3 | 57 |
| huile soja | 40 | 40 |
| Saindoux | 0 | 235 |
| sels minéraux | 35 | 62,5 |
| Vitamines | 10 | 12,5 |
| Cellulose | 50 | 62,5 |
| Choline | 2,3 | 2,3 |

### Sacrifice :

Au sacrifice, le poids des souris (Bwt), ainsi que le poids du foie, du tissu adipeux épidydymaire (TAE) et du tissu adipeux sous-cutané (TASC) sont mesurés.

Des cellules du tissu adipeux épidydymaire sont prélevées pour une numération et une analyse morphologique.

### Résultats

### Gain de poids

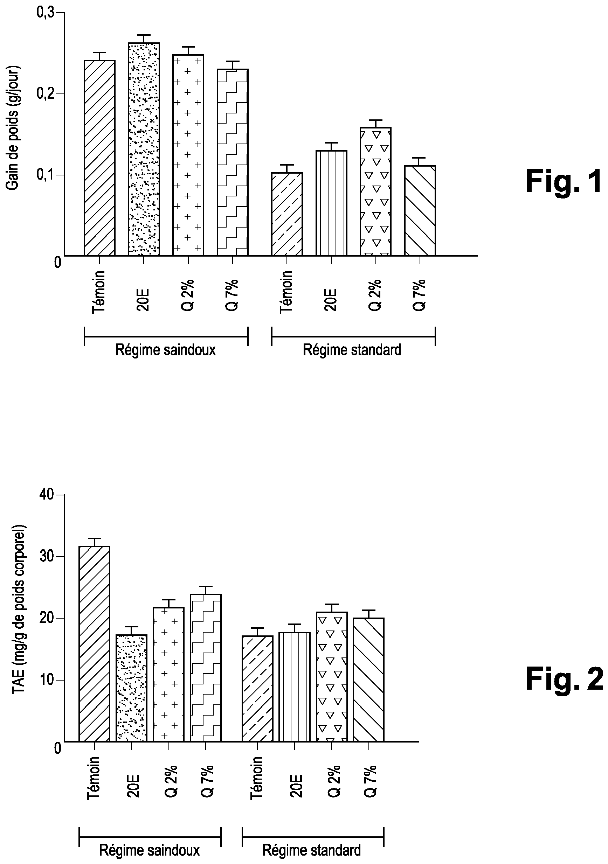

Sur la figure 1 est représenté un graphe montrant le gain de poids en g/jours des souris en fonction de leur régime et du traitement associé.

De manière attendue, on constate que les souris nourries au Saindoux ont un gain de poids plus important que celles qui n'ont pas reçu de Saindoux. Les souris soumises au régime Saindoux ont gagné en moyenne 250 mg/jour, et ce quel que soit le traitement. Par contre, le traitement quel qu'il soit, ne modifie pas significativement le gain de poids aussi bien à l'intérieur du groupe de souris nourries au Saindoux qu'au sein du groupe de souris nourries au régime témoin.

### Mesure de la masse grasse.

Sur la figure 2 est représenté un graphe montrant la quantité de masse grasse au niveau du tissu gonadal (%TAE) en fonction du régime et du traitement associé.

De manière attendue, on note une augmentation de l'adiposité plus importante chez les souris témoins nourries avec le régime Saindoux que chez les souris témoins nourries au régime normal.

Chez les souris ayant reçu un traitement, en association avec le régime saindoux, le traitement avec la molécule pure et les extraits à 2%, et 7% fait baisser l'adiposité respectivement de 50%, 30% et 20%.

Les souris ayant reçu un traitement par les extraits à 2% et 7% en association avec le régime témoin, présentent une adiposité légèrement supérieure à celle des souris témoins non traitées, mais cet effet n'est pas significatif. En revanche, le traitement par la molécule pure ne modifie pas l'adiposité des souris nourries avec le régime témoin.

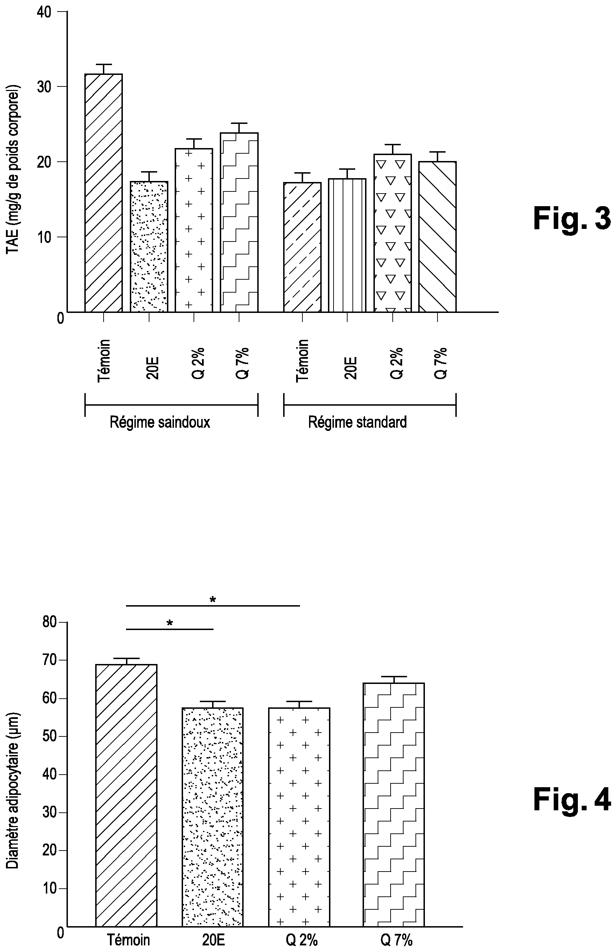

Sur la figure 3 est représenté un graphe montrant la quantité de masse grasse au niveau du tissu sous-cutané inguinal en fonction du régime et du traitement associé.

Les résultats sont comparables à ceux de l'adiposité au niveau du tissu gonadal. Chez les souris nourries au Saindoux, la diminution est cependant moins importante avec le traitement par les extraits à 2% et 7%. Chez les souris nourries au régime témoin, il n'y a aucun effet significatif des traitements.

### Mesure des adipocytes

Les cellules adipeuses, ou adipocytes, sont les cellules constitutives du tissu adipeux. Ces cellules comportent dans leur cytoplasme une gouttelette lipidique qui joue un rôle essentiel dans la synthèse des lipides et leur stockage. On peut penser que si le diamètre de ces cellules diminue, du fait donc de la diminution du diamètre de la gouttelette lipidique, cela aura un impact sur la quantité de masse grasse stockée.

Sur la figure 4 on peut voir l'effet du traitement sur le diamètre des adipocytes. Chez les souries nourries au régime Saindoux, le diamètre des adipocytes est hypertrophié par rapport aux souris témoins. Le traitement par la molécule pure et par l'extrait à 2% réduit la taille adipocytaire de manière significative. En revanche l'extrait à 7% n'a pas d'effet significatif sur le diamètre adipocytaire.

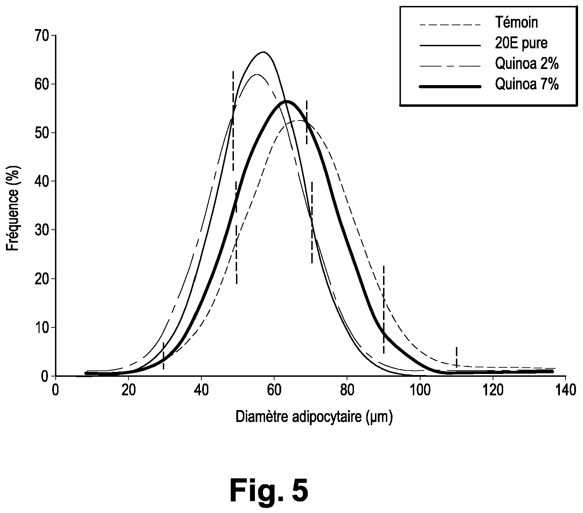

Sur la figure 5 sont représentés les effets du traitement sur la distribution des diamètres des adipocytes.

Les souris témoins nourries au Saindoux présentent une distribution avec une majorité des cellules ayant un diamètre important (jusqu'à 110 nm avec un mode d'environ 70). Les souris à qui on administre la phytoecdysone pure présentent une distribution avec un diamètre allant au maximum jusqu'à 90 nm, avec un mode de 60. Les souris à qui on administre l'extrait à 2% présentent une distribution similaire avec une majorité de cellules de petite taille. Les souris à qui on administre l'extrait à 7% présentent une distribution entre celle du témoin Saindoux et celle de l'extrait à 2%.

### Conclusion

L'administration de la molécule pure, comme celle de l'extrait à 2%, empêche un développement de l'obésité induite par un régime hyperlipidique et hypercalorique à base de Saindoux et est sans effet chez l'animal non obèse ayant un régime normal, c'est-à-dire ni hyper, ni hypocalorique. Cet effet est comparable sur le tissu gonadal et sur le tissu sous-cutané. L'extrait à 7% induit une légère adiposité (sous cutanée) en cas de régime témoin et présente une diminution de l'adiposité équivalente à celle de l'extrait à 2% en cas de régime Saindoux.

La comparaison des résultats de l'adiposité avec celle du gain de poids montre que les souris croissent et prennent du poids mais pas de masse grasse. Il y a vraisemblablement augmentation de la masse maigre musculaire.

### III] Exemple de formulation d'extrait de quinoa à 2% dans une forme galénique de type gélules

On ajoute 375 mg d'extrait de quinoa enrichi en phytoecdysones à hauteur de 2% en poids, à 75 mg de fécule de manioc. On introduit l'ensemble dans une gélule de type 600 mg. Une unique gélule permet à un patient d'ingérer 7,5 mg de phytoecdysones. La posologie préconisée, en cas de surcharge pondérale pouvant être diagnostiquée comme à risque chez un patient du point de vu des complications médicales et notamment pour l'installation du syndrome métabolique, est de 4 gélules par jour, de manière à ce que le patient ait un apport quotidien en phytoecdysones de 30 mg.

### Références

Báthori M, Pongrácz Z 2005 Phytoecdysteroids - from isolation to their effects on humans. Curr. Med. Chem. 12 153-172.
Chen Q, Xia Y, Qiu Z 2004 Use of ecdysterone in préparation of insulin résistance formulation. Application CN 1002-1686 20040113 (Chemical Abstracts 143 159564).
Chen Q, Xia Y, Qiu Z 2006 Effect of ecdysterone on glucose metabolism in vitro. Life Sci. 78 1108 - 1113.
Dini I, Tenore GC, Dini A 2005 Nutritional and antinutritional composition of Kancolla seeds : an interesting and underexploited andine food plant. Food Chem. 92 125-132.
Findeisen E 2004 Ecdysteroide in der menschlichen Nahrung. Ph.D. Thesis, University of Marburg (Germany).
Foster-Schubert KE, Cummings DE 2006 Emerging therapeutic stratégies for obesity. Endocrine Rev. 27, 779-793.
Isomaa B, Almgren P, Tuomi T, Forsén B, Lahti K, Nissén M, Taskinen MR, Groop L 2001 Cardiovascular morbidity and mortality associated with the metabolic syndrome. Diabetes Care 24, 683-689.
Khimiko IN, Mitrokin Yul, Efremova OI, Sidorenko LI 2000 The influence of ecdysterone on the biosynthesis of proteins and nucleic acids in mouse organs. Khim.-Farm. Zh. 34 3-5.
Klinzing Nielsen BK, Elgaard T, Jacobsen SE 2005 Feed additive derived from plant material originating from quinoa (Chenopodium quinoa). Patent application GB 2420066A.
Kuzmenko Al, Niki E, Noguchi N 2001 New functions of 20-hydroxyecdysone in lipid peroxidation. J. Oleo. Sci. 50, 497-506.
Lafont R, Dinan L 2003 Practical uses for ecdysteroids in mammals including humans: an update. J. Insect Sci. 3, pp. 30 (www.insectscience.org/3.7).
McWorther LS 2001 Biological complementary therapies: a focus on botanical products in diabetes. Diabetes Spectrum 14, 199-208.
Mironova VN, Kholodova, YuD, Skatchkova TF, Bonda OP, Datsenko ZM, Govseeva NN 1982 Hypocholesterolemic effects of phytoecdysones in rat experimental hypercholesterolemia. Vopr. Med. Khim. 28,101-105.
Muir AD, Paton D, Ballantyne K, Aubin AA 2002 Process for recovery and purification of saponins and sapogenins from quinoa (Chenopodium quinoa). Patent application United States Patent 6355249.
Otaka T, Uchiyama M, Okui S, Takemoto T, Hikino H, Ogawa S, Nishimoto N 1968 Stimulatory effect of insect metamorphosing steroids from Achyranthes and Cyathula on protein synthesis in mouse liver. Chem. Pharm. Bull. 16 2426-2429.
Pittle MH, Schmidt K, Ernst E 2005 Adverse events of herbal food supplements for body weight réduction : systematic review. Obesity Rev. 6, 93-111.
Purser DB, Baker SK. 1994. Ecdysones used to improve productivity of ruminants. PCT Int. Appl. WO 94 18,984, AU Appl. 93/7,397 (Chem. Abstr. 121 254587).
Rexrode K, Carey V, Hennekens CH, Walters EE, Colditz GA, Stampfer MJ, Willett WC, Manson JE 1998 Abdominal adiposity and coronary heart disease in women. JAMA 280, 1843-1848.
Saper RB, Eisenberg DM, Phillips RS 2004 Common dietary supplements for weight loss. Amer. Fam. Physician 70, 1731-1738.
Simon P, Koolman J 1989 Ecdysteroids in vertebrates : pharmacological aspects. In: Koolman J (ed), Ecdysone, from chemistry to mode of action. Georg Thieme Verlag, Stuttgart, pp 254-259.
Syrov VN 2000 Comparative experimental investigations of the anabolic activity of ecdysteroids and steranabols. Pharm. Chem. J. 34 193-197.
Syrov, VN, Khushbaktova ZA, Abzalova MKh, Sultanov MB 1983 On the hypolipidemic and antiatherosclerotic action of phytoecdysteroids. Dokl. Akad. Nauk Uzb. SSR (9) 44-45.
Syrov VN, Nabiev AN, Sultanov MB 1986 The effect of phytoecdysteroids on the bile secretion function of the liver in normal rats and in animais with experimental hepatitis. Farmakol. Toksikol. 49 100-103.
Takahashi H, Nishimoto K 1992 Antidiabetic agents containing ecdysterone or inokosterone. Jpn Kokai Tokkyo Koho J.P. 04,125,135 [92 124,135]. (Chem. Abstr. 117: 84874b).
Uchiyama M, Ogawa S. 1970. Hypoglycemic formulations containing insect hormones. Application JP 19690506 (Chem. Abstr. 74 : 24985).
Unger RH 2003 Minireview: weapons of lean body mass destruction: the role of ectopic lipids in the metabolic syndrome. Endocrinology 144, 5159-5165.
Wasan KM, Looije NA 2005 Emerging pharmacological approaches in the treatment of obesity. J. Pharm. Pharmaceut. Sci. 8, 259-271.
Yang C, Zhang G, Liu X, Wang C 2001 Oral antidiabetic compositions containing β-ecdysone from Cyanothis arachnoides*.* Appl. CN-2000-10637/20000612 (Chem. Abstr. 135: 127188).
Zhu N, Kikusaki H, Vastano BC, Nakatani N, Karwe MV, Rosen RT, Ho CT 2001 Ecdysteroids of quinoa seeds (Chenopodium quinoa Willd.). J. Agric. Food Chem. 49, 2576-2578.

## Revendications

1. Composition à base de phytoecdysones apportée sous forme de 20-hydroxyecdysone pure ou sous forme d'extrait de plantes enrichi en phytoecdysone, pour son utilisation chez le mammifère dans la prévention et/ou le traitement du syndrome métabolique chez le mammifère en surpoids ou obèse par diminution de la masse grasse au niveau de la ceinture abdominale.

2. Composition à base de phytoecdysones pour son utilisation selon la revendication 1, **caractérisée en ce que** l'extrait de plantes provient du quinoa.

3. Composition à base de phytoecdysones pour son utilisation selon la revendication 1 ou 2, dans laquelle 85% à 90% des phytoecdysones correspondent à de la 20-hydroxyecdysone.

4. Composition à base de phytoecdysones pour son utilisation selon l'une des revendications 1 à 3, en tant qu'aliment ou complément alimentaire.

5. Composition à base de phytoecdysones pour son utilisation selon l'une des revendications 1 à 4, en tant que composition médicinale.

6. Composition à base de phytoecdysones pour son utilisation selon l'une des revendications 1 à 5, **caractérisé en ce qu'**elle est sous une forme permettant une posologie comprise entre 20 mg/jour et 30 mg/jour de phytoecdysones.

7. Composition à base de phytoecdysones pour son utilisation selon l'une des revendications 1 à 6, **caractérisé en ce que** les plantes utilisées pour former l'extrait sont choisies parmi les plantes alimentaires riches en phytoecdysones et **en ce que** ledit extrait comporte au moins 1 % en poids de phytoecdysones.

8. Composition à base de phytoecdysones pour son utilisation selon l'une des revendications 1 à 6, **caractérisé en ce que** l'extrait est un extrait de quinoa sans saponines comportant entre 1% et 7% en poids de phytoecdysones.

9. Composition à base de phytoecdysones pour son utilisation selon l'une des revendications 1 à 7, **caractérisé en ce que** l'extrait comporte 2% en poids de phytoecdysones.

## Patentansprüche

1. Zusammensetzung auf der Basis von Phytoecdysonen, beigebracht in Form von reinem 20-Hydroxyecdyson oder in Form von mit Phytoecdyson angereichertem Pflanzenextrakt, für ihre Verwendung beim Säuger bei der Vorbeugung und/oder der Behandlung des metabolischen Syndroms beim übergewichtigen oder adipösen Säuger durch Verringerung der Fettmasse im Abdominalgürtel.

2. Zusammensetzung auf der Basis von Phytoecdysonen für ihre Verwendung nach Anspruch 1, **dadurch gekennzeichnet, dass** der Pflanzenextrakt von Quinoa stammt.

3. Zusammensetzung auf der Basis von Phytoecdysonen für ihre Verwendung nach Anspruch 1 oder 2, wobei 85 % bis 90 % der Phytoecdysone dem 20-Hydroxyecdyson entsprechen.

4. Zusammensetzung auf der Basis von Phytoecdysonen für ihre Verwendung nach einem der Ansprüche 1 bis 3 als Nahrung oder Nahrungsergänzung.

5. Zusammensetzung auf der Basis von Phytoecdysonen für ihre Verwendung nach einem der Ansprüche 1 bis 4 als medizinische Zusammensetzung.

6. Zusammensetzung auf der Basis von Phytoecdysonen für ihre Verwendung nach einem der Ansprüche 1 bis 5, **dadurch gekennzeichnet, dass** sie in einer Form ist, die eine Dosierung erlaubt, die zwischen 20 mg/Tag und 30 mg/Tag an Phytoecdysonen liegt.

7. Zusammensetzung auf der Basis von Phytoecdysonen für ihre Verwendung nach einem der Ansprüche 1 bis 6, **dadurch gekennzeichnet, dass** die für die Bildung des Extrakts verwendeten Pflanzen aus den an Phytoecdysonen reichen Nahrungspflanzen ausgewählt sind und dadurch, dass der Extrakt mindestens 1 Gew.-% an Phytoecdysonen aufweist.

8. Zusammensetzung auf der Basis von Phytoecdysonen für ihre Verwendung nach einem der Ansprüche 1 bis 6, **dadurch gekennzeichnet, dass** der Extrakt ein Quinoaextrakt ohne Saponine ist, der zwischen 1 und 7 Gew.-% an Phytoecdysonen aufweist.

9. Zusammensetzung auf der Basis von Phytoecdysonen für ihre Verwendung nach einem der Ansprüche 1 bis 7, **dadurch gekennzeichnet, dass** der Extrakt 2 Gew.-% an Phytoecdysonen aufweist.

## Claims

1. Composition based on phytoecdysones provided in the form of pure 20-hydroxyecdysone or in the form of phytoecdysone-enriched plant extract, for use in mammals in the prevention and/or treatment of metabolic syndrome in overweight or obese mammals by reducing abdominal body fat mass.

2. Composition based on phytoecdysones for use according to claim 1, **characterised in that** the plant extract comes from quinoa.

3. Composition based on phytoecdysones for use according to claim 1 or 2, wherein 85% to 90% of the phytoecdysones correspond to 20-hydroxyecdysone.

4. Composition based on phytoecdysones for use according to one of claims 1 to 3, as a food or dietary supplement.

5. Composition based on phytoecdysones for use according to one of claims 1 to 4, as a medicinal composition.

6. Composition based on phytoecdysones for use according to one of claims 1 to 5, **characterised in that** it is in a form enabling a dosage between 20 mg/day and 30 mg/day of phytoecdysones.

7. Composition based on phytoecdysones for use according to one of claims 1 to 6, **characterised in that** the plants used to form the extract are chosen among phytoecdysone-rich food plants and **in that** said extract includes at least 1% by weight of phytoecdysones.

8. Composition based on phytoecdysones for use according to one of claims 1 to 6, **characterised in that** the extract is a saponin-free quinoa extract including between 1% and 7% by weight of phytoecdysones.

9. Composition based on phytoecdysones for use according to one of claims 1 to 7, **characterised in that** the extract includes 2% by weight of phytoecdysones.
